# EUROPEAN PATENT APPLICATION

(11) **EP 3 693 726 A1**
(43) Date of publication of application: **12.08.2020**
(21) Application number: 18864174.0
(22) Date of filing: 28.09.2018
(51) Int. Cl.: G01N 21/27, G02B 21/00

(54) **IMAGING DEVICE, METHOD FOR ACTUATING IMAGING DEVICE, AND IMAGING CONTROL PROGRAM**

(30) Priority: 03.10.2017 JP 2017193651
(71) Applicant: Fujifilm Corporation, Minato-ku Tokyo 106-8620 (JP)
(72) Inventor: MUROOKA Takashi, Ashigarakami-gun Kanagawa 258-8538 (JP)
(74) Representative: Klunker IP Patentanwälte PartG mbB
(86) International application number: PCT/JP2018/036395
(87) International publication number: WO 2019/069823

(57) **Abstract**

In an imaging device, and an operation method and an imaging control program of the imaging device, it is possible to acquire a captured image by suppressing a decrease in image quality caused by droplets attached to a container. Before a heating unit heats a container in which an observation target is housed, and an imaging unit performs main imaging for the observation target housed in the container, a pre-measurement unit measures brightness of light transmitting through or reflected by the container. In a case where a value of the brightness measured by a pre-measurement unit is smaller than a standard value set in advance, the control unit causes the imaging unit to perform the main imaging after droplets attached to the container are removed by increasing a temperature of the container by the heating unit or by keeping the temperature of the container constant by the heating unit.

## Description

### BACKGROUND OF THE INVENTION

### 1. Field of the Invention

The present invention relates to an imaging device that images an observation target housed in a container, and an operation method and an imaging control program of the imaging device.

### 2. Description of the Related Art

Pluripotent stem cells such as embryonic stem (ES) cells and induced pluripotent stem (iPS) cells have the ability to differentiate into cells of various tissues, and are attracting attention as being applicable to regenerative medicine, drug development, elucidation of diseases, and the like.

A method of imaging pluripotent stem cells such as ES cells and iPS cells or cells induced to differentiate by using a microscope device or the like, and evaluating the differentiation status of the cells on the basis of the characteristics of the image has been proposed.

Generally, in case of imaging the cells by using the microscope device, imaging conditions are set in advance and imaging is performed under the set imaging conditions. However, in the container where the cultured pluripotent stem cells are housed, due to various factors such as human error, wrong type of container, or changes in the amount or density of culture solution caused by fogging of the container, that is, droplets attached to the container, evaporation and the like, the set imaging conditions may not be necessarily optimal conditions at the time of imaging, in some cases.

Thus, JP2013-228361A discloses a technique of acquiring a preliminary captured image by performing preliminary imaging, performing imaging again (main imaging) by setting the light intensity distribution of irradiation light such that the amount of incident light is larger in a portion with lower luminance in the preliminary captured image, and thereby reducing density unevenness of an image caused by the irradiation light.

### SUMMARY OF THE INVENTION

On the other hand, generally, pluripotent stem cells such as ES cells and iPS cells are housed in a culture vessel such as a well plate, and are cultured in a state where the environmental temperature and environmental humidity are controlled, in an incubator. In a case where the cultured pluripotent stem cells are imaged, the culture vessel is moved from the incubator to the microscope device.

However, at the time of moving the culture vessel from the incubator to the microscope device, dew condensation occurs due to the differences in temperature and humidity between the incubator and the outside air, and thus droplets may be attached to the bottom surface of the culture vessel, the lid of the culture vessel, and the like to cause fogging in the culture vessel, in some cases. In a case where the cells are imaged in a state where droplets are attached to the culture vessel, a focal position of light that has passed through the cells is shifted due to the droplets, so that there is a problem in that a blurred image is obtained. Even at the time of performing an autofocus control, for example, in a case where the position of the bottom surface of the culture vessel is detected by a laser displacement sensor, the light intensity of the laser displacement sensor is decreased to be lower than an actual value due to the fogging, so that appropriate autofocus cannot be obtained, and thus there is still a problem in that a blurred image is obtained.

The invention is made in view of the above-described circumstances, and an object of the invention is to provide an imaging device which can acquire a captured image by suppressing a decrease in image quality due to droplets attached to a container, and an operation method and an imaging control program of the imaging device.

An imaging device of the invention comprises an imaging unit that images an observation target housed in a container; a heating unit that heats the container in which the observation target is housed; a pre-measurement unit that measures brightness of light transmitting through or reflected by the container before main imaging by the imaging unit; and a control unit that, in a case where a value of the brightness measured by the pre-measurement unit is lower than a standard value set in advance, causes the imaging unit to perform the main imaging after droplets attached to the container are removed by increasing a temperature of the container by the heating unit or by keeping the temperature of the container constant by the heating unit.

In the invention, the "container" is collectively referred to as a container including a lid in a case where the container is covered with the lid.

In the invention, the "main imaging" means imaging performed in a case where a captured image that a user desires is acquired, and the "pre-measurement" means pre-measurement performed before the main imaging.

In the imaging device of the invention, the pre-measurement unit may irradiate a bottom surface of the container with light and measure light intensity of reflected light which is reflected by the bottom surface.

In this case, the pre-measurement unit may be a laser displacement sensor.

In the imaging device of the invention, the pre-measurement unit may measure a pixel value in a transmission image which is acquired by imaging the observation target housed in the container using the imaging unit.

In the invention, the "light intensity" and the "pixel value" correspond to brightness.

In the imaging device of the invention, the control unit may determine that the droplets attached to the container are removed in a case where a time set in advance elapses from a start of heating of the container by the heating unit.

In the invention, as a time point at which the heating of the container by the heating unit is started, in a case where the heating unit is not being operated, a time point at which the heating unit is operated is regarded as a time point at which the heating is started, and in a case where the heating unit is operated in advance, one of a time point at which the container is installed on the imaging device, a time point at which there is an input indicating that the heating is started by the user, and a time point at which the control unit determines that the value of the brightness is smaller than the standard value set in advance can be adopted. The time point at which the heating is started may be set and changed by the user.

In the imaging device of the invention, the control unit may increase the time set in advance as the value of the brightness is lower.

The imaging device of the invention further comprises a stage on which the container in which the observation target is housed is installed, in which the container in which the observation target is housed may be housed in an incubator before being installed on the stage, and the heating unit may heat the container up to a temperature in the incubator.

In the imaging device of the invention, the heating unit may be constituted by a heat glass, or may be constituted by an incubator.

An operation method of an imaging device of the invention is an operation method of an imaging device comprising an imaging unit, a heating unit, a pre-measurement unit, and a control unit, the operation method comprising heating a container in which an observation target is housed, using the heating unit; measuring brightness of light transmitting through or reflected by the container before main imaging is performed for the observation target housed in the container by the imaging unit, using the pre-measurement unit; and in a case where a value of the brightness measured by the pre-measurement unit is lower than a standard value set in advance, causing the imaging unit to perform the main imaging after droplets attached to the container are removed by increasing a temperature of the container by the heating unit or by keeping the temperature of the container constant by the heating unit, using the control unit.

The operation method of the imaging device of the invention may be provided as a program to be executed by a computer.

Another imaging device of the invention is an imaging device comprising an imaging unit that images an observation target housed in a container, a heating unit that heats the container in which the observation target is housed, a pre-measurement unit that measures brightness of light transmitting through or reflected by the container before main imaging by the imaging unit, a memory that stores a command for a computer to execute, and a processor that executes the stored command, in which in a case where a value of the brightness measured by the pre-measurement unit is lower than a standard value set in advance, the processor causes the imaging unit to perform the main imaging after droplets attached to the container are removed by increasing a temperature of the container by the heating unit or by keeping the temperature of the container constant by the heating unit.

With the imaging device, and the operation method and the imaging control program of the imaging device of the invention, before the heating unit heats the container in which the observation target is housed, and the imaging unit performs main imaging for the observation target housed in the container, the pre-measurement unit measures the brightness of the light transmitting through or reflected by the container. In a case where the value of the brightness measured by the pre-measurement unit is lower than the standard value set in advance, since the control unit causes the imaging unit to perform main imaging after the droplets attached to the container are removed by increasing the temperature of the container by the heating unit or by keeping the temperature of the container constant by the heating unit, it is possible to acquire a captured image by suppressing a decrease in image quality caused by the droplets attached to the container.

### BRIEF DESCRIPTION OF THE DRAWINGS

Fig. 1 is a diagram illustrating a schematic configuration of a microscope device of a first embodiment in a microscope observation system of the embodiment.
Fig. 2 is a schematic diagram illustrating a configuration of an image-forming optical system.
Fig. 3 is a perspective diagram illustrating a construction of a stage.
Fig. 4 is a block diagram illustrating a configuration of the microscope observation system of the embodiment.
Fig. 5 is a schematic diagram illustrating an aspect in which droplets are attached to a culture vessel.
Fig. 6 is a schematic diagram illustrating an aspect in which droplets are attached to a culture vessel with a lid.
Fig. 7 is a flowchart illustrating a process of the first embodiment which is performed in the microscope observation system of the embodiment.
Fig. 8 is a flowchart illustrating a process of a second embodiment which is performed in the microscope observation system of the embodiment.
Fig. 9 is a diagram illustrating a schematic configuration of a microscope device of the second embodiment in the microscope observation system.

### DESCRIPTION OF THE PREFERRED EMBODIMENTS

Hereinafter, a microscope observation system 1 in which an imaging device of a first embodiment of the invention is used will be described in detail with reference to the drawings.

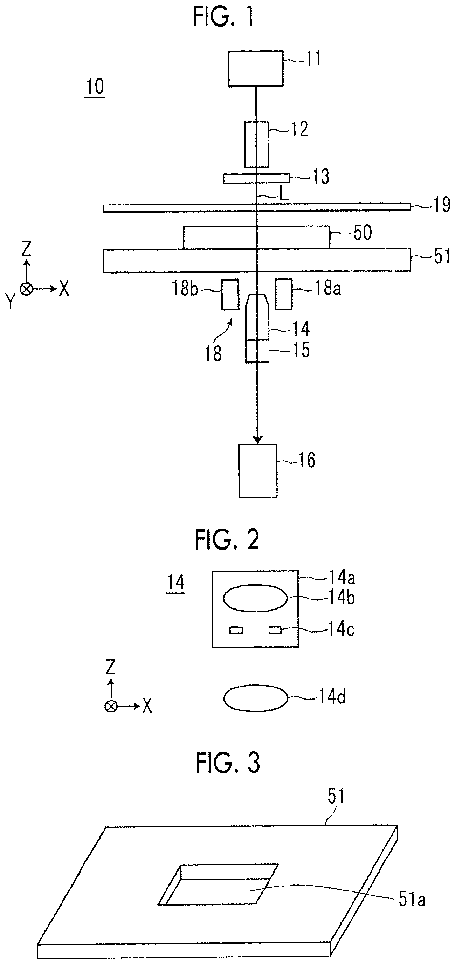

Fig. 1 is a diagram illustrating a schematic configuration of a microscope device 10 in the microscope observation system 1 of the first embodiment. In the embodiment, the microscope observation system 1 includes the microscope device 10 and a microscope control device 20 which will be described below, and the microscope observation system 1 corresponds to the imaging device of the invention.

The microscope device 10 captures a phase contrast image of cultured cells as an observation target. Specifically, as illustrated in Fig. 1, the microscope device 10 comprises a white light source 11 that emits white light, a condenser lens 12, a slit plate 13, an image-forming optical system 14, an image-forming optical system driving unit 15, an imaging unit 16, a detection unit 18, a stage 51, and a heating unit 19.

The slit plate 13 is obtained such that a ring-shaped slit transmitting white light is provided to a light shielding plate that shields white light emitted from the white light source 11, and the white light passes through the slit to form ring-shaped illumination light L.

Fig. 2 is a diagram illustrating a detailed configuration of the image-forming optical system 14. As illustrated in Fig. 2, the image-forming optical system 14 comprises a phase contrast lens 14a and an image-forming lens 14d. The phase contrast lens 14a comprises an objective lens 14b and a phase plate 14c. The phase plate 14c is obtained such that a phase ring is formed on a transparent plate transparent for the wavelength of the illumination light L. The size of the slit of the slit plate 13 has a conjugate relationship with the phase ring of the phase plate 14c.

In the phase ring, a phase film for shifting the phase of the incident light by 1/4 wavelength, and a neutral density filter for dimming the incident light are formed in a ring shape. Direct light incident on the phase ring passes through the phase ring so that the phase of the direct light is shifted by 1/4 wavelength and the brightness is weakened. Meanwhile, most of diffracted light diffracted by the observation target passes through the transparent plate of the phase plate 14c so that the phase and brightness thereof are not changed.

The phase contrast lens 14a having the objective lens 14b is moved in an optical axis direction of the objective lens 14b by the image-forming optical system driving unit 15 illustrated in Fig. 1. In the embodiment, the optical axis direction of the objective lens 14b is the same as a Z direction (vertical direction). The autofocus control is performed by the movement of the phase contrast lens 14a in the Z direction, and the contrast of the phase contrast image to be captured by the imaging unit 16 is adjusted.

In addition, a configuration in which the magnification of the phase contrast lens 14a can be changed may be adopted. Specifically, the image-forming optical system 14 or the phase contrast lens 14a having different magnifications may be configured to be exchangeable. The exchange of the phase contrast lens 14a or the image-forming optical system 14 may be automatically performed or may be manually performed by a user.

The image-forming optical system driving unit 15 comprises an actuator such as a piezoelectric element, and is driven on the basis of a control signal output from a control unit 22 which will be described below. The image-forming optical system driving unit 15 is configured to pass, as it is, the phase contrast image which has passed through the phase contrast lens 14a. In addition, the configuration of the image-forming optical system driving unit 15 is not limited to the piezoelectric element, and may be any configuration as long as the configuration moves the phase contrast lens 14a in the Z direction, and other known configurations can be used.

The phase contrast image which has passed through the phase contrast lens 14a and the image-forming optical system driving unit 15 is incident on the image-forming lens 14d, and the image-forming lens 14d forms the phase contrast image on the imaging unit 16.

The imaging unit 16 comprises an imaging element that receives an image of an observation target S which is formed by the image-forming lens 14d to image the observation target S and outputs the phase contrast image as an observation image. As the imaging element, it is possible to use a charge coupled device (CCD) image sensor, a complementary metal oxide semiconductor (CMOS) image sensor, and the like. As the imaging element, an imaging element provided with red, green, and blue (RGB) color filters may be used or a monochrome imaging element may be used.

The detection unit 18 detects the position of a culture vessel 50, which is installed on the stage 51, in the Z direction (vertical direction). Specifically, the detection unit 18 comprises a first displacement sensor 18a and a second displacement sensor 18b. The first displacement sensor 18a and the second displacement sensor 18b are provided side by side in an X direction illustrated in Fig. 1 with the phase contrast lens 14a interposed therebetween. In the embodiment, the first displacement sensor 18a and the second displacement sensor 18b are a laser displacement meter (laser displacement sensor), irradiate the culture vessel 50 with laser light, and detect the reflected light thereof to detect the position of the bottom surface of the culture vessel 50 in the Z direction. In the embodiment, the detection unit 18 corresponds to a pre-measurement unit of the invention, and measures the light intensity of the reflected light. The detection unit 18 is used as the pre-measurement unit of the embodiment, but the invention is not limited to the detection unit 18, and for example, a laser displacement sensor may be provided to the microscope device 10 separately from the detection unit 18. As the laser displacement sensor, a specular reflection optical system measuring instrument can be used. The invention is not limited to the laser displacement sensor, and for example, a confocal sensor can be used.

Positional information indicating the position of the culture vessel 50 in the Z direction, which is detected by the detection unit 18, is output to the control unit 22, and the control unit 22 controls the image-forming optical system driving unit 15 on the basis of the input positional information to perform the autofocus control.

The stage 51 is provided between the slit plate 13, and the phase contrast lens 14a and the detection unit 18. On the stage 51, the culture vessel 50 in which the cells as the observation target are housed is installed.

As the culture vessel 50, it is possible to use a petri dish, dishes or a well plate in which a plurality of wells are arranged. In the embodiment, the well plate in which a plurality of wells are arranged is used as the culture vessel 50. In addition, as the cells housed in the culture vessel 50, there are pluripotent stem cells such as iPS cells and ES cells; nerve, skin, myocardial, and liver cells induced to differentiate from stem cells; and skin, retina, myocardial, blood, nerve, and organ cells removed from the human body.

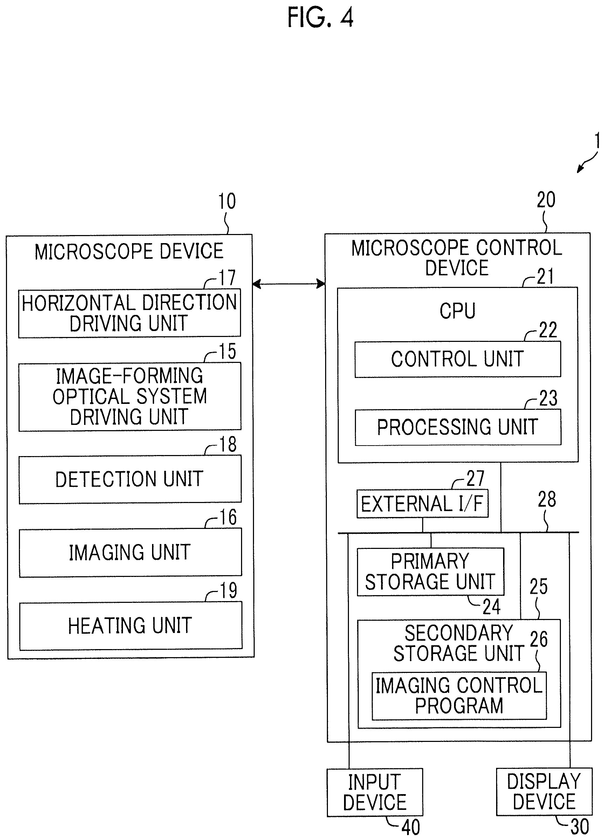

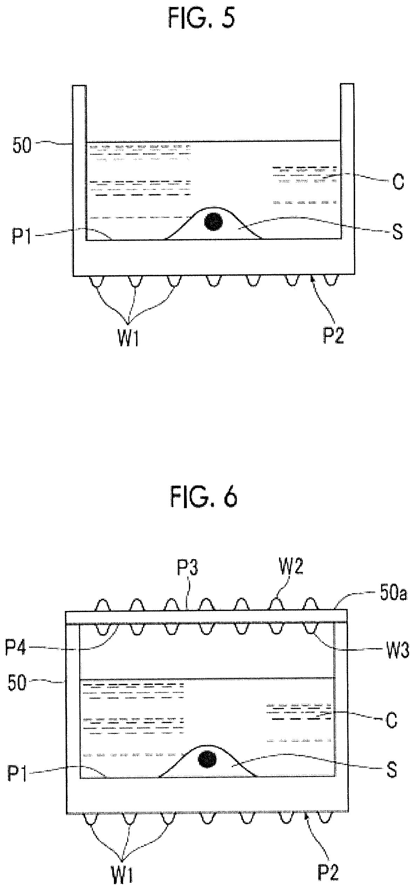

The stage 51 is moved in the X direction and a Y direction, which are orthogonal to each other, by a horizontal direction driving unit 17 (refer to Fig. 4). The X direction and the Y direction are directions orthogonal to each other on a surface parallel to an observation target installation surface PI, and the Z direction is a direction orthogonal to the X direction and the Y direction. The observation target installation surface P1 is a boundary surface between the bottom of the culture vessel 50 and the cells as the observation target (refer to Figs. 5 and 6).

Fig. 3 is a diagram illustrating an example of the stage 51. A rectangular opening 51a is formed in the center of the stage 51. The culture vessel 50 is installed on a member forming the opening 51a, and the phase contrast image of the cells in the culture vessel 50 passes through the opening 51a.

The heating unit 19 heats the culture vessel 50 in which the cells as the observation target are housed. In the embodiment, the heating unit 19 is constituted by a heat glass, and the heat glass is installed on the stage 51 so as to cover a movable range of the stage 51 in the X direction and the Y direction in which the stage 51 is moved. In addition, the heating unit 19 performs temperature setting so as to increase the temperature of the culture vessel 50 up to the temperature in the incubator in which the cells as the observation target S housed in the culture vessel 50 are housed at the time of being cultured, that is, the incubator in which the culture vessel 50 is housed before being installed on the stage 51. In the embodiment, the temperature is set to, for example, 35 degrees. The control unit 22 controls on/off of the operation of the heating unit 19.

Next, a configuration of the microscope control device 20 that controls the microscope device 10 will be described. Fig. 4 is a block diagram illustrating the configuration of the microscope control device 20 of the first embodiment. For the microscope device 10, a partial configuration controlled by each unit of the microscope control device 20 is illustrated as a block diagram.

The microscope control device 20 is constituted by a computer comprising a central processing unit (CPU) 21, a primary storage unit 24, a secondary storage unit 25, an external interface (I/F) 27, and the like.

The CPU 21 comprises the control unit 22 and a processing unit 23, and controls the entire microscope observation system 1. The primary storage unit 24 is a volatile memory that is used as a work area and the like at the time of execution of various programs. Examples of the primary storage unit 24 include a random access memory (RAM). The secondary storage unit 25 is a non-volatile memory that stores various programs, various parameters, and the like in advance, and an embodiment of an imaging control program 26 of the invention is installed in the secondary storage unit 25. The imaging control program 26 is executed by the CPU 21 so that the control unit 22 and the processing unit 23 function. Examples of the secondary storage unit 25 include an electrically erasable programmable read-only memory (EEPROM) or a flash memory. The external I/F 27 manages transmission and reception of various kinds of information between the microscope device 10 and the microscope control device 20. The CPU 21, the primary storage unit 24, and the secondary storage unit 25 are connected to a bus line 28. In addition, the external I/F 27 is also connected to the bus line 28.

The imaging control program 26 is distributed by being recorded in a recording medium such as a digital versatile disc (DVD) and a compact disc read only memory (CD-ROM), and is installed to a computer from the recording medium. Otherwise, the imaging control program 26 may be stored in a storage device of a server computer connected to a network or a network storage in a state of being accessible from the outside, and downloaded to a computer in response to the request from the outside to be installed.

In the above description, a case in which a general-purpose computer functions as the microscope control device 20 has been described, but the microscope control device 20 may be implemented by a dedicated computer. The dedicated computer may be firmware that executes a program recorded in a non-volatile memory such as a built-in read only memory (ROM) or a flash memory. Further, a dedicated circuit such as an application specific integrated circuit (ASIC) or a field programmable gate arrays (FPGA) that permanently stores a program for executing at least a part of the functions of the microscope control device 20 may be provided. Alternatively, a program command stored in the dedicated circuit may be combined with a program command executed by a general-purpose CPU programmed to use the program of the dedicated circuit. As described above, the program command may be executed in any combination of hardware configurations of the computer.

The control unit 22 controls the image-forming optical system driving unit 15 on the basis of the information of the position of the culture vessel 50 in the Z direction which is detected by the detection unit 18 as described above. Then, the objective lens 14b of the image-forming optical system 14 is moved in the optical axis direction by the drive of the image-forming optical system driving unit 15 so that the autofocus control is performed. In addition, the control unit 22 controls the drive of the horizontal direction driving unit 17 so that the stage 51 is moved in the X direction and the Y direction. The horizontal direction driving unit 17 is constituted by an actuator having a piezoelectric element or the like.

In the embodiment, the stage 51 is moved in the X direction and the Y direction under the control of the control unit 22, and the image-forming optical system 14 is two-dimensionally scanned in the culture vessel 50, thereby capturing phase contrast images at respective observation positions by image-forming optical system 14. That is, a phase contrast image is captured for each of a plurality of imaging regions (view field) divided in one well.

In addition, the control unit 22 functions as a display control unit which causes a display device 30 to display one composite phase contrast image generated by composing the phase contrast images captured at respective observation positions by the microscope device 10.

In the embodiment, in a case where the light intensity measured by the detection unit 18 is lower than a standard value set in advance, the control unit 22 causes the imaging unit 16 to perform main imaging after the droplets attached to the culture vessel 50 are removed by increasing the temperature of the culture vessel 50 by the heating unit 19 or by keeping the temperature of the culture vessel 50 constant by the heating unit 19. Here, the main imaging means imaging performed at the time of acquiring a phase contrast image that the user desires.

Here, the droplets attached to the culture vessel 50 will be described. Fig. 5 is a schematic diagram illustrating an aspect in which droplets W1 are attached to the culture vessel 50, and Fig. 6 is a schematic diagram illustrating an aspect in which the droplets W1, W2, and W3 are attached to the culture vessel 50 with a lid 50a. The cells as the observation target S housed in the culture vessel 50 are cultured in a state where the environmental temperature and the environmental humidity are managed, in the incubator (not illustrated). In a case where the image of the cultured cells is captured, the culture vessel 50 is moved from the incubator to the microscope device 10.

In a case where the culture vessel 50 is extracted from the incubator, dew condensation occurs due to the differences in temperature and humidity between the incubator and the outside air, and thus, as illustrated in Fig. 5, the droplets W1 are attached to a bottom surface P2 to cause fogging in the culture vessel 50. Further, as illustrated in Fig. 6, in a case where the culture vessel 50 is covered with the lid 50a, droplets W2 are attached to an upper surface P3 of the lid 50a and droplets W3 are attached to a lower surface P4 of the lid 50a, in some cases. In a case where the droplets W1, W2, and W3 are attached to the bottom surface P2 of the culture vessel 50, and the upper surface P3 and the lower surface P4 of the lid 50a, when the detection unit 18 detects the position of the bottom surface P2 of the culture vessel 50 in the autofocus control, the light intensity of the detection unit 18 is decreased to be lower than an actual value, that is, than a case where the droplets W1 are not attached, due to the droplets W1 attached to the bottom surface P2 in particular, so that an appropriate autofocus control cannot be performed.

In the microscope observation system of the embodiment, before the main imaging by the imaging unit 16, pre-measurement of irradiating the culture vessel 50 with laser light using the detection unit 18 and measuring the light intensity of the reflected light is performed. Then, the control unit 22 determines whether the light intensity measured by the detection unit 18 is lower than the standard value set in advance, and in a case where the light intensity is determined to be lower than the standard value, on the assumption that the droplets W1 are attached, the droplets W1 are removed, and then the imaging unit 16 captures a phase contrast image of the observation target S. For the standard value for the light intensity, a value at which the phase contrast image is preferable is examined in advance, the light intensity of the culture vessel 50 in a state where the droplets W1 are not attached is measured, and the measured value is stored as the standard value in the secondary storage unit 25.

In the embodiment, the control unit 22 causes the imaging unit 16 to perform main imaging after the droplets W1 attached to the bottom surface P2 are removed by increasing the temperature of the culture vessel 50 by the heating unit 19 or by keeping the temperature of the culture vessel 50 constant by the heating unit 19. The temperature of the culture vessel 50 is not always increased, for example, in a case where the measurement time is long, the temperature in the incubator and the temperature of the culture vessel 50 become the same temperature, and thus the temperature of the culture vessel 50 may be hardly increased. The temperature of the incubator is controlled to be always constant, and even in the embodiment, the culture vessel 50 is controlled by the control unit 22 to have the same temperature as the temperature in the incubator using the heating unit 19. Hunting of up and down of about ±0.5°C may occur.

Specifically, in a case where a time set in advance elapses from the start of the heating of the culture vessel 50 by the heating unit 19, the control unit 22 determines that there is no droplets W1 attached to the bottom surface P2 of the culture vessel 50. In the embodiment, the heating unit 19 is operated with the power turned on in advance. Accordingly, a time point at which the heating of the culture vessel 50 by the heating unit 19 is started is regarded as a time point at which the light intensity of the reflected light is determined to be lower than the standard value by the control unit 22, and in a case where, for example, 60 seconds elapse from the time point at which the light intensity is determined to be smaller than the standard value, the control unit 22 determines that the droplets W1 are removed. In the embodiment, a case where 60 seconds elapse is used, but the invention is not limited thereto, and the elapse time can be appropriately set and changed by the user. In addition, the control unit 22 may set the elapse time longer as the light intensity of the reflected light is lower. As the light intensity is lower, the possibility that many droplets W1 are attached is increased, that is, the degree of fogging is increased, and therefore, it is possible to reliably remove the droplets W1 by increasing the elapse time. In this case, a table in which the light intensity and the elapse time are associated with each other can be stored in the secondary storage unit 25.

The time point at which the heating of the culture vessel 50 by the heating unit 19 is started is not limited to the above-described time point, and for example, in a case where the heating unit 19 is operated in advance, a time point at which the culture vessel 50 is installed on the stage 51 may be used, or a time point at which the user operates an input device 40, which will be described below, to input that the heating is started may be used. In a case where the heating unit 19 is not being operated, a time point at which the heating unit 19 is operated may be used as the time point at which the heating is started.

In the embodiment, before the imaging unit 16 performs main imaging for the observation target S housed in the culture vessel 50, the pre-measurement unit, that is, the detection unit 18 measures the light intensity of the light reflected by the bottom surface of the culture vessel 50, and in a case where the measured light intensity is lower than the standard value set in advance, since the control unit 22 causes the imaging unit 16 to perform main imaging after the droplets W1 attached to the bottom surface P2 of the culture vessel 50 are removed by increasing the temperature of the culture vessel 50 by the heating unit 19 or by keeping the temperature of the culture vessel 50 constant by the heating unit 19, it is possible to acquire a captured image by suppressing a decrease in image quality caused by the droplets W1 attached to the culture vessel 50.

Next, returning to Fig. 4, the processing unit 23 performs various kinds of processing, such as gamma correction, luminance/color difference conversion, and compression processing, on image signals acquired by the imaging unit 16. In addition, the processing unit 23 outputs the image signals, which are obtained through the various kinds of processing, to the control unit 22 for each frame at a specific frame rate. In addition, the processing unit 23 generates one composite phase contrast image by composing the phase contrast images captured at respective observation positions R by the microscope device 10.

The input device 40 and the display device 30 are connected to the microscope control device 20 through the bus line 28.

The display device 30 displays the generated composite phase contrast image under the control of the control unit 22, and comprises a liquid crystal display or the like. The display device 30 is constituted by a touch panel and may also be used as the input device 40.

The input device 40 comprises a mouse, a keyboard, and the like, and receives various setting inputs from the user. In the embodiment, the input device 40 receives a setting input such as an instruction for changing the magnification of the phase contrast lens 14a and an instruction for changing the moving speed of the stage. The input device 40 also receives a setting input such as an instruction for changing the above-described elapse time. Further, the input device 40 can receive an input indicating that the temperature of the culture vessel 50 is started to be increased by the heating unit 19.

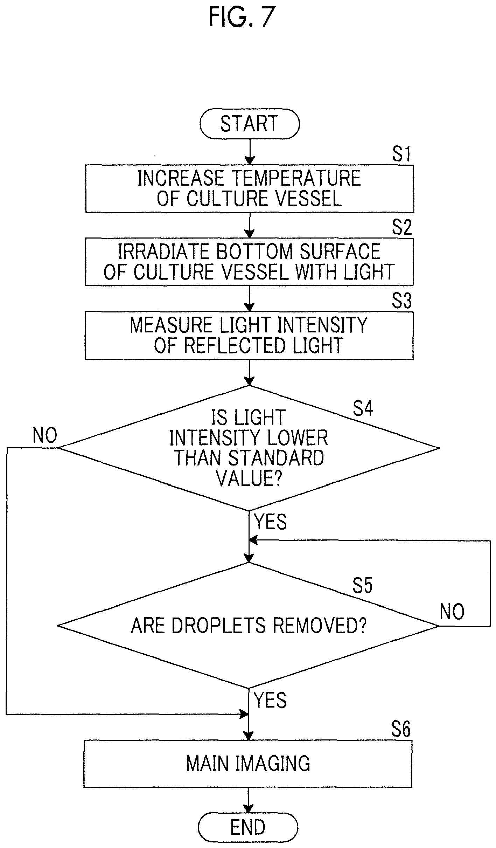

Next, a process performed by the microscope observation system 1 of the embodiment will be described. Fig. 7 is a flowchart illustrating a process performed in the microscope observation system I of the embodiment.

First, in a state where the power of the heating unit 19 is turned on and the heating unit 19 is being operated, the culture vessel 50 in which the observation target S is housed is extracted from the incubator and is installed on the stage 51, and the heating unit 19 heats the culture vessel 50 to increase the temperature of the culture vessel 50 (step S1).

Next, the detection unit 18 irradiates the bottom surface P2 of the culture vessel 50 with laser light (step S2), and measures the light intensity of the reflected light thereof (step S3).

Next, in a case where the control unit 22 determines that the measured light intensity is lower than the standard value (step S4; YES), and in a case where the control unit 22 determines that the droplets W1 attached to the bottom surface P2 of the culture vessel 50 are removed, that is, in a case where 60 seconds elapse from the start of the heating of the culture vessel 50 by the heating unit 19 (step S5; YES), the control unit 22 causes the imaging unit 16 to perform main imaging (step S6). In a case where the control unit 22 determines that the droplets W1 attached to the bottom surface P2 of the culture vessel 50 are not removed, for example, in a case where 60 seconds do not elapse (step S5; NO), the process of step S5 is repeated until the control unit 22 determines that the droplets W1 are removed, that is, until the 60 seconds elapse.

On the other hand, in a case where in step S4, the control unit 22 determines that the measured light intensity is equal to or greater than the standard value (step S4; NO), the CPU 21 causes the process to proceed to step S6, and the control unit 22 causes the imaging unit 16 to perform main imaging (step S6).

In this manner, imaging by the microscope observation system 1 is performed. The phase contrast images of the observation target S which are captured at respective observation positions R are composed by the processing unit 23 so that one composite phase contrast image is generated, and the generated composite phase contrast image is displayed on the display device 30 under the control of the control unit 22.

With the microscope observation system of the embodiment, the before heating unit 19 heats the culture vessel 50 in which the observation target S is housed, and the imaging unit 16 performs main imaging for the observation target S housed in the culture vessel 50, the detection unit 18 measures the light intensity of the light reflected by the culture vessel 50. In a case where the light intensity measured by the detection unit 18 is lower than the standard value set in advance, since the control unit 22 causes the imaging unit 16 to perform main imaging after the droplets W1 attached to the bottom surface P2 of the culture vessel 50 are removed by increasing the temperature of the culture vessel 50 by the heating unit 19 or by keeping the temperature of the culture vessel 50 constant by the heating unit 19, it is possible to acquire a captured image by suppressing a decrease in image quality caused by the droplets W1 attached to the bottom surface P2 of the culture vessel 50.

Next, a microscope observation system in which an imaging device of a second embodiment of the invention is used will be described. The microscope observation system of the embodiment has the same configuration of the microscope observation system 1 of the above-described embodiment illustrated in Fig. 1, and only the pre-measurement unit is different. Therefore, only the pre-measurement unit will be described below, and the description of other configurations is omitted.

In the embodiment, before the main imaging by the imaging unit 16, the pre-measurement unit performs pre-measurement of measuring a pixel value in a transmission image, that is, a phase contrast image which is acquired by imaging the observation target S housed in the culture vessel 50 by the imaging unit 16.

In a case where the culture vessel 50 is covered with the lid 50a and the droplets are attached to the culture vessel 50, the light which is emitted from the white light source 11 and is incident on the culture vessel 50 through the slit plate 13 is scattered by at least one of the droplets W2 attached to the upper surface P3 or the droplets W3 attached to the lower surface P4 of the lid 50a of the culture vessel 50. Therefore, the focal position of the transmitted light which is transmitted through the observation target S is shifted, and thus the phase contrast image acquired by the imaging by the imaging unit 16 becomes a blurred image.

In the embodiment, before the main imaging by the imaging unit 16 is performed, the pre-measurement unit performs pre-measurement of measuring a pixel value in a phase contrast image which is acquired by the control unit 22 causing the imaging unit 16 to perform imaging, that is, pre-imaging. Then, the control unit 22 determines whether the pixel value measured by the pre-measurement unit is lower than the standard value set in advance, and in a case where the control unit 22 determines that the pixel value is lower than the standard value, on the assumption that the droplets W1, W2, and W3 are attached, the droplets W1, W2, and W3 are removed, and then the control unit 22 causes the imaging unit 16 to capture a phase contrast image of the observation target S again. That is, the main imaging is performed. For the standard value for the pixel value, a value at which the phase contrast image is preferable is examined in advance, the pixel value of the phase contrast image acquired by imaging the observation target S in a state where the droplets W1, W2, and W3 are not attached is measured, and the measured value is stored as the standard value in the secondary storage unit 25. Specifically, the user checks that there is no fogging in the culture vessel 50 of the same type (for example, the same maker model number) containing a medium of the type to be used in the experiment, and then the entire culture vessel 50 is spatially evenly measured and averaged. For example, in a case where the culture vessel 50 is a plate having six wells, three points in each well are measured so that an average value of the pixel values of a total of 18 points is stored as the standard value. The sum of the pixel values may be stored as the standard value.

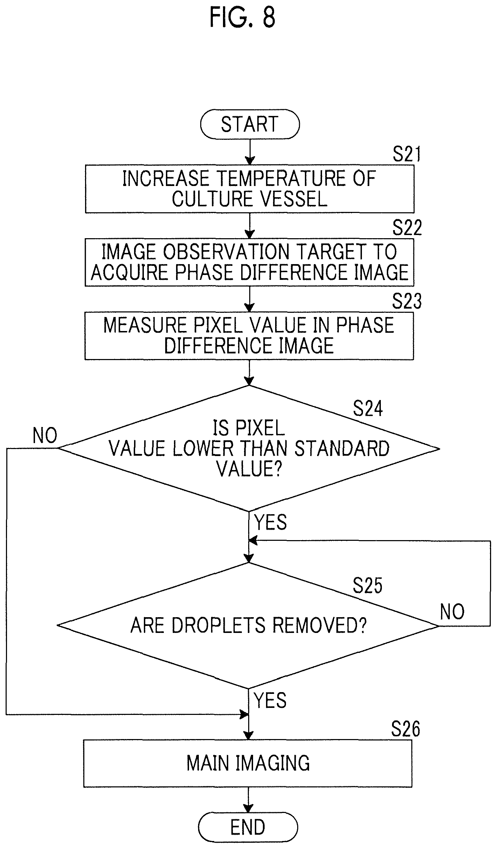

Next, a process performed by the microscope observation system of the embodiment will be described. Fig. 8 is a flowchart illustrating a process performed in the microscope observation system of the embodiment.

First, in a state where the power of the heating unit 19 is turned on and the heating unit 19 is being operated, the culture vessel 50 in which the observation target S is housed is extracted from the incubator and is installed on the stage 51, and the heating unit 19 heats the culture vessel 50 to increase the temperature of the culture vessel 50 (step S21).

Next, the control unit 22 causes the imaging unit 16 to capture the observation target S housed in the culture vessel 50 to acquire a phase contrast image (transmission image) (step S22). Then, the pre-measurement unit measures the pixel value in the phase contrast image (step S23). In the embodiment, specifically, the average value of the pixel values is calculated as described above.

Next, in a case where the control unit 22 determines that the average value of the measured pixel values is lower than the standard value (step S24; YES), the control unit 22 determines whether the droplets W1, W2, and W3 attached to the culture vessel 50 are removed. In a case where the control unit 22 determines that the droplets are removed, that is, in a case where 60 seconds elapse from the start of an increase in temperature of the culture vessel 50 by the heating unit 19 (step S25; YES), the control unit 22 causes the imaging unit 16 to perform main imaging (step S26). In addition, in a case where the control unit 22 determines that the droplets W1, W2, and W3 attached to the culture vessel 50 are not removed, for example, in a case where 60 seconds do not elapse (step S25; NO), the process of step S25 is repeated until the control unit 22 determines that the droplets W1, W2, and W3 are removed, that is, until the 60 seconds elapse.

On the other hand, in a case where in step S24, the control unit 22 determines that the average value of the measured pixel values is equal to or greater than the standard value (step S24; NO), the CPU 21 causes the process to proceed to step S26, and the control unit 22 causes the imaging unit 16 to perform main imaging (step S26).

In this manner, imaging by the microscope observation system is performed. The phase contrast images of the observation target S which are captured at respective observation positions are composed by the processing unit 23 so that one composite phase contrast image is generated, and the generated composite phase contrast image is displayed on the display device 30 under the control of the control unit 22.

With the microscope observation system of the embodiment, before the heating unit 19 heats the culture vessel 50 in which the observation target S is housed and the imaging unit 16 performs main imaging for the observation target S housed in the culture vessel 50, the control unit 22 causes the imaging unit 16 to perform pre-imaging for the observation target S to acquire a phase contrast image, and the pre-measurement unit measures pixel values in the acquired phase contrast image and calculates an average value of the pixel values. In a case where the average value of the pixel values measured by the pre-measurement unit is lower than the standard value set in advance, since the control unit 22 causes the imaging unit 16 to perform main imaging after the droplets W1, W2, and W3 attached to the culture vessel 50 are removed by increasing the temperature of the culture vessel 50 by the heating unit 19, it is possible to acquire a captured image by suppressing a decrease in image quality caused by the droplets W1, W2, and W3 attached to the culture vessel 50.

The microscope observation system of the embodiment is configured as described above, but the invention is not limited thereto, and can be appropriately modified in a range without departing from the scope of the invention.

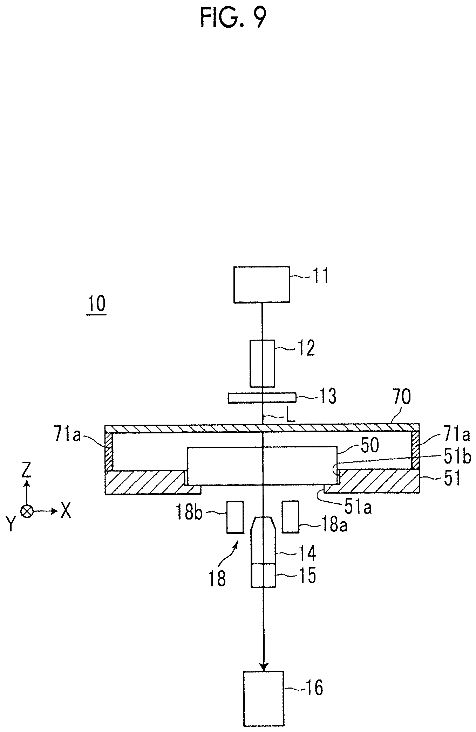

In the embodiment, the heating unit 19 is constituted by the heat glass, but may be constituted by a small incubator 70 formed in a crown shape covering the stage 51, as illustrated in Fig. 9. In this case, it is possible to remove the droplets by decreasing the humidity or increasing the temperature in the small incubator 70. The small incubator 70 can be constituted by a heat glass having a frame 71a, and the culture vessel 50 can be constituted by a container with a heat panel. In this case, a holding part 51b constituted by a hole for holding the culture vessel 50 is provided on the upper side of the opening 51a of the stage 51, and the peripheral edge portion of the bottom of the culture vessel 50 is caught by the edge of the holding part 51b so that the culture vessel 50 is held. Specifically, a thermobox manufactured by TOKAI HIT Co., Ltd. may be used.

The microscope observation system of the embodiment performs the autofocus control, but may be configured no to perform the autofocus control.

In the embodiment, the invention is applied to a phase contrast microscope, but the invention is not limited to the phase contrast microscope, and can be applied to other microscopes such as a differential interference microscope and a bright field microscope.

Hereinafter, effects of the embodiment will be described.

Before a container in which an observation target is housed is heated and main imaging is performed for the observation target housed in the container, brightness of light transmitting through or reflected by the container is measured. In a case where the value of the measured brightness is smaller than a standard value set in advance, since main imaging is performed after droplets attached to the container are removed by increasing the temperature of the container by a heating unit or by keeping the temperature of the container constant by the heating unit, it is possible to acquire a captured image by suppressing a decrease in image quality caused by the droplets attached to the container.

### Explanation of References

1: microscope observation system
10: microscope device
11: white light source
12: condenser lens
13: slit plate
14: image-forming optical system
14a: phase contrast lens
14b: objective lens
14c: phase plate
14d: image-forming lens
15: image-forming optical system driving unit
16: imaging unit
17: horizontal direction driving unit
18: detection unit
19: heating unit
20: microscope control device
21: CPU
22: control unit
23: processing unit
24: primary storage unit
25: secondary storage unit
26: imaging control program
27: external I/F
28: bus line
30: display device
40: input device
50: culture vessel
50a: lid
51: stage
51a: opening
51b: holding part
70: small incubator
L: illumination light
C: culture solution
P1: observation target installation surface
P2: bottom surface
P3: upper surface
P4: lower surface
S: observation target
W1: droplets
W2: droplets
W3: droplets

## Claims

1. An imaging device comprising:
an imaging unit that images an observation target housed in a container;
a heating unit that heats the container in which the observation target is housed;
a pre-measurement unit that measures brightness of light transmitting through or reflected by the container before main imaging by the imaging unit; and
a control unit that, in a case where a value of the brightness measured by the pre-measurement unit is lower than a standard value set in advance, causes the imaging unit to perform the main imaging after droplets attached to the container are removed by increasing a temperature of the container by the heating unit or by keeping the temperature of the container constant by the heating unit.

2. The imaging device according to claim 1,
wherein the pre-measurement unit irradiates a bottom surface of the container with light and measures light intensity of reflected light which is reflected by the bottom surface.

3. The imaging device according to claim 1,
wherein the pre-measurement unit measures a pixel value in a transmission image which is acquired by imaging the observation target housed in the container using the imaging unit.

4. The imaging device according to any one of claims 1 to 3,
wherein the control unit determines that the droplets attached to the container are removed in a case where a time set in advance elapses from a start of heating of the container by the heating unit.

5. The imaging device according to claim 4,
wherein the control unit increases the time set in advance as the value of the brightness is lower.

6. The imaging device according to any one of claims 1 to 5, further comprising:
a stage on which the container in which the observation target is housed is installed,
wherein the container in which the observation target is housed is housed in an incubator before being installed on the stage, and
the heating unit heats the container up to a temperature in the incubator.

7. The imaging device according to claim 2,
wherein the pre-measurement unit is a laser displacement sensor.

8. The imaging device according to any one of claims 1 to 7,
wherein the heating unit is constituted by a heat glass.

9. The imaging device according to any one of claims 1 to 7,
wherein the heating unit is constituted by an incubator.

10. An operation method of an imaging device comprising an imaging unit, a heating unit, a pre-measurement unit, and a control unit, the operation method comprising:
heating a container in which an observation target is housed, using the heating unit;
measuring brightness of light transmitting through or reflected by the container before main imaging is performed for the observation target housed in the container by the imaging unit, using the pre-measurement unit; and
in a case where a value of the brightness measured by the pre-measurement unit is lower than a standard value set in advance, causing the imaging unit to perform the main imaging after droplets attached to the container are removed by increasing a temperature of the container by the heating unit or by keeping the temperature of the container constant by the heating unit, using the control unit.

11. An imaging control program for controlling imaging of an imaging device comprising an imaging unit that images an observation target housed in a container, a heating unit that heats the container in which the observation target is housed, and a pre-measurement unit that measures brightness of light transmitting through or reflected by the container before main imaging by the imaging unit, the imaging control program causing a computer to function as:
control means for, in a case where a value of the brightness measured by the pre-measurement unit is lower than a standard value set in advance, causing the imaging unit to perform the main imaging after droplets attached to the container are removed by increasing a temperature of the container by the heating unit or by keeping the temperature of the container constant by the heating unit.
